# EUROPEAN PATENT SPECIFICATION

(11) **EP 2 272 551 B1**
(45) Date of publication and mention of the grant of the patent: **06.06.2012**
(21) Application number: 10157638.7
(22) Date of filing: 24.03.2010
(51) Int. Cl.: A61M 1/02, A61M 39/28

(54) **Device for controlling the supply of red blood cells to a patient in a transfusion line**
Vorrichtung zur Steuerung der Versorgung von mit roten Blutkörperchen einem Patienten in einem Transfusionsschlauch
Dispositif pour contrôler l'administration de globules rouges à un patient dans une ligne de transfusion

(30) Priority: 09.04.2009 IT MI20090579
(43) Date of publication of application: 12.01.2011
(73) Proprietor: Eurosets S.r.l., 41036 Medolla (MO) (IT)
(72) Inventor: Fontanili, Paolo, 42015, Correggio RE (IT); Balugani, Fabio, 41032, Cavezzo MO (IT); Paratelli, Ruggero, 41036, Medolla MO (IT)
(74) Representative: Zoli, Filippo

(56) References cited:
- EP-A- 0 593 102
- EP-A- 1 065 495
- WO-A-2008/002135
- FR-A- 2 625 320
- US-A- 3 215 394
- US-A- 4 697 785
- US-A- 4 807 676
- US-A- 5 282 982

## Description

The present invention relates to a device for controlling the supply of red blood cells to a patient in a transfusion line.

It is known that blood transfusion to patients is a widespread practice particularly in surgery and is performed by means of a line which comprises a blood collection tank which is connected, by means of a duct, to a tank for infusion of the blood to the patient, or directly to the patient.

Blood transfusion can be performed with autologous blood, i.e., blood drawn from the patient himself, and in this case one speaks of autotransfusion, or with homologous blood, i.e., blood from a blood bank; in the first case there are three types of autotransfusion, designated by terms that need no comment: one in fact speaks of autotransfusion with prebanked blood, of intraoperative autotransfusion, and of postoperative autotransfusion.

In all cases gravity sedimentation of the blood occurs in the collection tank and thus over time a stratification is created which is determined by the different specific weight of the components, in which in the lower portion of the tank there are red blood cells at a high concentration and, rising upwards, there are a decrease to substantially no concentration of said red blood cells and the presence of different components, such as white blood cells, platelets, fats.

Hereinafter, the term "red blood cells" shall be used to designate the material constituted by red blood cells at high concentration that is present in the lower portion of the collection tank, and the term "supernatant" shall be used to designate the material that collects above the red blood cells.

It is important to clarify immediately that the infusion to the patient relates only to the red blood cells, whereas the supernatant is eliminated.

In order to optimize the methods of implementation of this operation, EPA 09154425.4 by the same Applicant discloses a device which comprises means adapted to detect the concentration of red blood cells in the blood collection tank by means of a detection performed on the tank proper or on the duct in output from such tank, and which are adapted to actuate fastening means which are adapted to selectively close and open the duct in output from the collection tank, so as to block or respectively allow the passage of the liquid flow through it.

Of course, at the beginning of the transfusion the device keeps the duct that exits from the blood collection tank in the open position with consequent passage of red blood cells and, when such device detects in the blood conveyed by such duct a red blood cell concentration that is considered to be the lowest acceptable for infusion to the patient, such duct is closed, consequently blocking the supernatant, which is thus prevented from reaching the patient.

The described device has proved to give very good results, and continuing studies have allowed improvement of the features of the present invention which has the aim of optimizing, with a particularly simple structure, the mode of operation of the device.

Blood separation systems with blood composition detectors and controlled delivery clamps are disclosed in documents EP 0593102 A1 and FR 2625320 A1.

This aim is achieved by a device for controlling the supply of red blood cells to a patient in a transfusion line, according to the invention, characterized in that it comprises the features cited in the appended claims.

Further characteristics and advantages of the invention will become better apparent from the description of a preferred but not exclusive embodiment of the device according to the invention, illustrated by way of non-limiting example in the accompanying drawings, wherein:
Figure 1 is a perspective view of a transfusion line comprising the device according to the invention;
Figure 2 is a perspective view of the device according to the invention with the door open;
Figure 3 is a perspective view of the device according to the invention with the door closed and with the blood conveyance duct in the closure position;
Figures 4 and 5 are respectively sectional views taken along the lines IV-IV and V-V of Figure 3;
Figures 6 and 7 are the same sectional views, but with the blood conveyance duct in the open position;
Figures 8, 9, 10 are respectively sectional views, taken along the line VIII-VIII and along the line IX-IX of Figure 3, and along the line X-X of Figure 2.

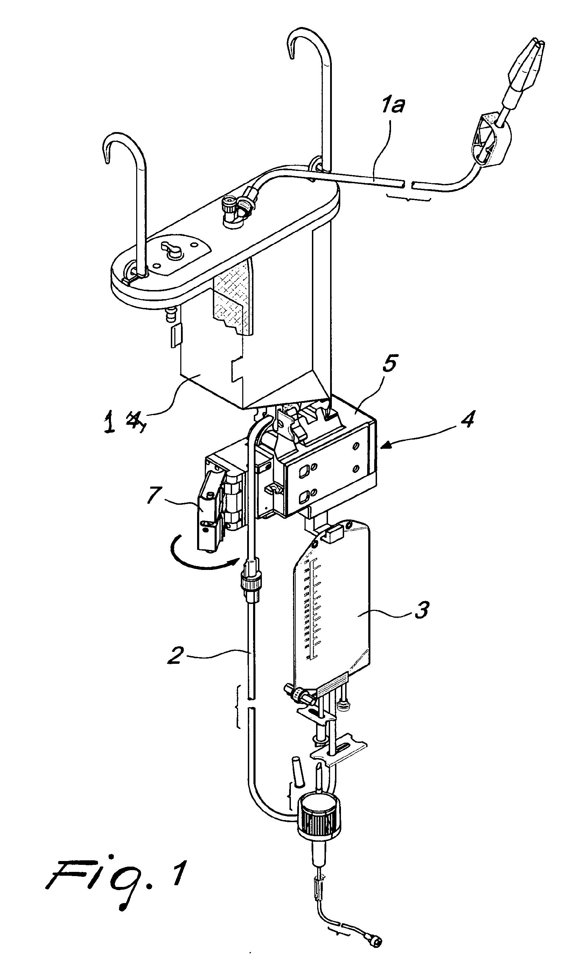

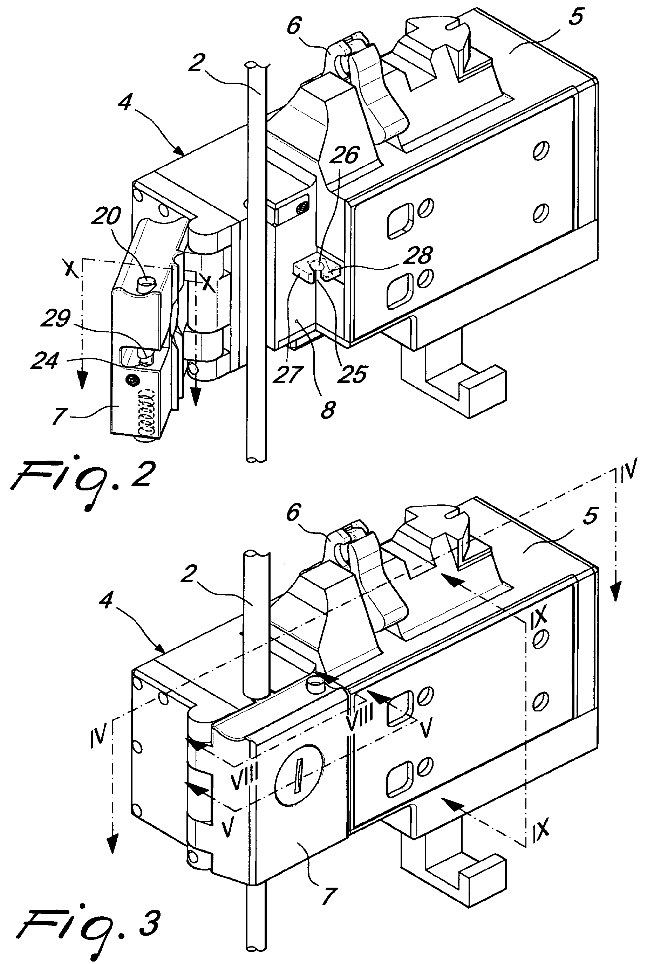

With reference to figure 1, which illustrates a blood transfusion line, the numeral 1 designates a blood collection tank, which can be of the type that comprises a mechanical supernatant separator, which reaches it through a duct 1a, connected by means of a duct 2 to a tank 3 for infusion to a patient; a device 4 according to the invention is installed on the duct 2 and is now described with reference to Figures 2 to 10.

The device 4 comprises a body 5, which has a spring clip for engaging the overlying tank, and is provided with a door 7 which is pivoted thereto and can move, along the arrow shown in Figure 1, between an open position shown in Figures 1 and 2, which allows to associate the device with the duct 2, and an active closure position that is visible in Figure 3; a microswitch 8 is pressed by the door 7 in such position, and operation is thus allowed.

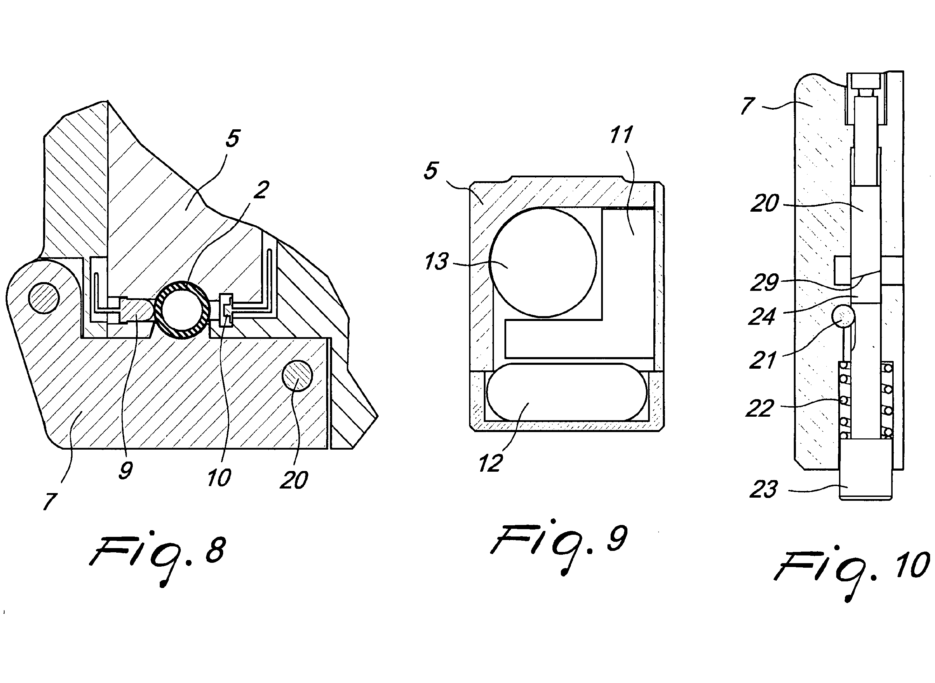

The body 5 accommodates, as shown in Figure 8, the means adapted to detect the concentration of red blood cells in the blood conveyed by the duct 2, which comprise a source 9 of a luminous radiation that is arranged so that said radiation passes through the duct 2, exiting from it at a receiver 10. Since the optical density of the blood is directly proportional to the concentration of red blood cells, according to an important feature of the invention the degree of absorption that the radiation undergoes in passing through the duct 2 is measured, thereby determining said concentration.

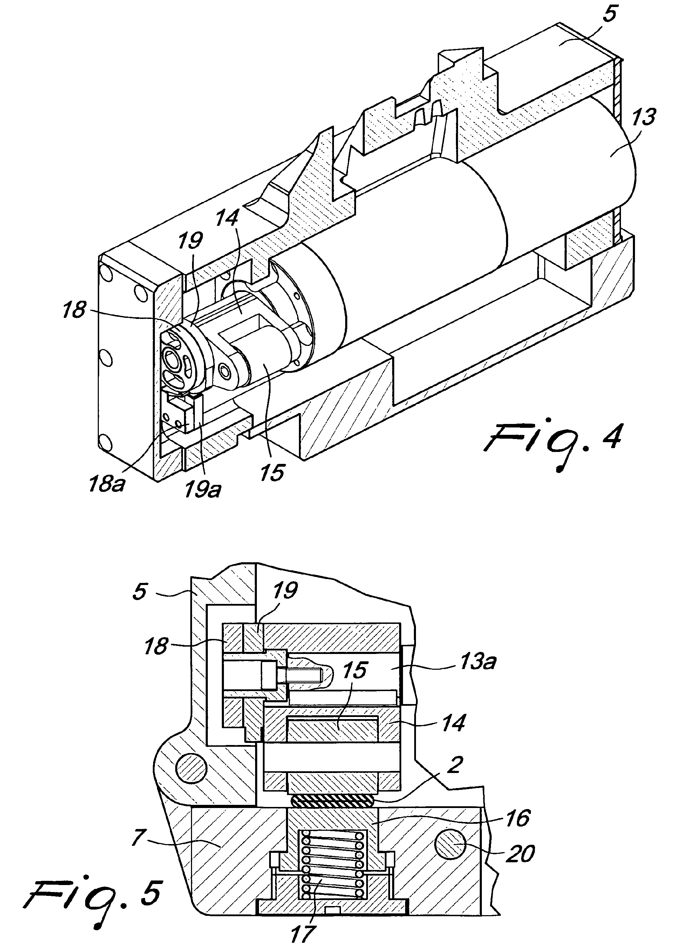

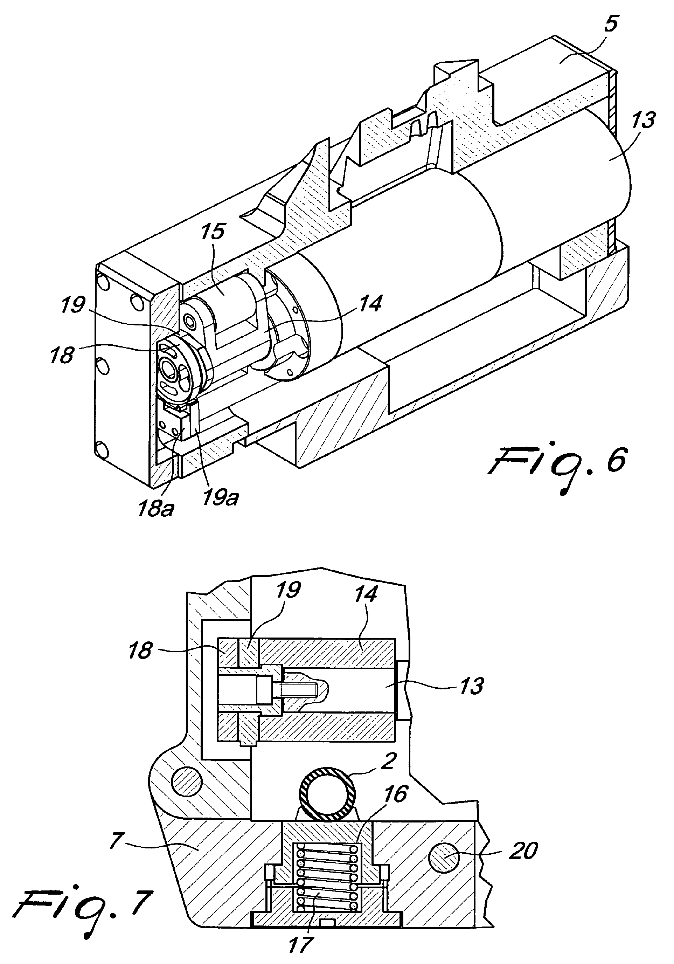

For this purpose, the source 9 and the receiver 10 are connected to a management card 11, which is accommodated within the body above a battery 12 that powers the device; such card and such battery are visible in Figure 9 but not shown in Figures 4 and 6 for graphical reasons.

When the described detection means, connected to the management card 11, detect in the blood conveyed by the duct 2 a red blood cell concentration that is considered to be the lowest acceptable for infusion to the patient, a signal is sent from the card 11 to means, which are now described in detail and which cause the closure of the duct 2.

Such means comprise, according to a further important feature of the invention, an electric motor 13, on an output shaft 13a of which an eccentric support 14 is keyed, a roller 15 being associated with said support and being designed to move between two stroke limit positions.

The first stroke limit position is shown in Figures 4 and 5; therein, the roller 15 compresses the duct 2 against an abutment 16 provided with an elastic element 17 until it is closed completely; the second stroke limit position is visible in Figures 6 and 7; therein, the roller 15 is not in contact with the duct 2, which is therefore in an open condition.

The numerals 18 and 19 further designate two fixed cams, which are fixed in an adjustable position on the output shaft 13a of the electric motor 13 and, by operating in combination with microswitches 18a, 19a, constitute the stroke limiters of the motion of said motor.

The door 7 is provided with means for locking in the closed position, which comprise a rod 20, which can slide axially, any rotation being prevented by a timing pin 21, in a seat formed in said door 7, and is kept, by the action of a spring 22, in the lower stroke limit position in which it protrudes by means of a button 23 from the lower face of the door 7.

The rod 20 is provided, in an intermediate position, with a face 24, which is adapted to enter snugly a notch 25 for accessing a seat 26 designed to accommodate the rod 20, which is formed in a plate 27 jointly connected to the body 5.

The notch 25 is flanked by an inclined plane 28, which is adapted to make contact with an inclined plane 29 that is present on the rod 20 at the upper edge of the face 24, and thus during the closing motion of the door 7, while the face 24 passes through the notch 25, the rod 20 is lifted, with loading of the spring 22, which causes at the appropriate time the snap insertion of the rod 20 in the seat 26, with consequent locking of the door 7.

To reopen such door it is sufficient to push the rod 20 upwardly by operating the button 23.

The described invention is susceptible of numerous modifications and variations, all of which are within the scope of the appended claims; all the details may further be replaced with other technically equivalent elements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device for controlling the supply of red blood cells to a patient in a transfusion line, said transfusion line comprising a blood collection tank (1) connected by means of a duct (2) to a tank (3) for infusion to the patient or directly to the patient, and said device (4) being provided with detection means (9, 10) that are adapted to detect the concentration of red blood cells in the blood contained in the collection tank (1) by means of a detection performed on said tank (1) or on the duct (2) that connects said collection tank (1) to the tank (3) for infusion to the patient or directly to the patient, and that are adapted to actuate fastening means (14, 15, 16 and 17) that are adapted selectively to close and open said duct (2) in output from the collection tank (1) so as to block or respectively allow the passage of the liquid flow through it, said detection means (9, 10) adapted to detect the concentration of red blood cells in the blood contained in the collection tank (1) being adapted to measure the degree of absorption of a luminous radiation that passes through the blood, the device (4) further comprising a body (5) provided with a seat that allows to associate said device (4) with the duct (2) in output from the blood collection tank (1), **characterized in that** said detection means (9, 10) adapted to detect the concentration of red blood cells in the flow of blood conveyed by said duct (2) are inserted in said body (5), are connected to a management card (11) that is accommodated within said body (5) and comprise a source (9) of a luminous radiation that is arranged so that said radiation passes through said duct (2) and exits from it at a receiver (10), said body (5) further comprising inside it said fastening means that comprise an electric motor (13) controlled by the management card (11) and adapted to actuate an eccentric support (14) keyed on the output shaft of the motor (13) and which supports a roller (15) between a first stroke limit position, in which the roller (15) compresses the duct (2) against an abutment (16) until it causes its complete closure, and a second stroke limit position, in which the roller (15) is out of contact with said duct (2), which therefore is in the open condition.

2. The device according to claim 1, **characterized in that** the body (5) comprises a door (7) that can move between an open position, which allows to associate said body (5) with the duct (2) in output from the blood collection tank (1), and a closed operating position.

3. The device according to claim 1 or 2, **characterized in that** the roller (15) associated with the eccentric support (14) actuated by the electric motor (13) compresses the duct (2) for conveying the blood against an elastic abutment (17).

4. The device according to one or more of the preceding claims, **characterized by** the presence of a stroke limiter for the motion of the electric motor for actuating the eccentric roller support (14) which comprises two cams (18, 19) that are associated adjustably with the output shaft (13a) of the motor (13) and operate in combination with microswitches (18a, 19a) associated with the management card (11) comprised within the body (5) of the device.

5. The device according to one or more of the claims 2-4, **characterized in that** said door (7) is hinged to the body of the device and is provided with means for locking in a closure position, which comprise a rod (20) that can slide axially within a seat that is formed in the door (7) and is maintained by the action of elastic means (22) in the lower stroke limit position, in which it protrudes by means of a button (23) from the lower face of said door (7), said rod (20) being provided, in an intermediate position, with a recess (24) that is adapted to be inserted snugly in an access notch (25) for accessing a seat (26) designed to accommodate said rod (20) formed in a plate (27) that is jointly connected to said body (5), said notch (25) being flanked by an inclined plane (28) that is adapted to come into contact with a corresponding inclined plane (29) that is provided on the rod (20) at the upper edge of the recess (24), so as to lift the rod (20) during the closing motion of the door (7) at the notch (25) up to snap insertion in its seat (26) produced by the action of the elastic means (22), with consequent locking of the door (7).

## Patentansprüche

1. Vorrichtung zur Steuerung der zuführung roter Blutkörperchen zu einem Patienten über eine Transfusionsleitung, wobei die Transfusionsleitung ein Blutsammelbehältnis (1), das mittels einer Kanals (2) mit einem Behältnis (3) zur Infusion an den Patienten bzw. direkt mit dem Patienten verbunden ist, umfasst, wobei die Vorrichtung (4) Erfassungsmittel (9, 10) aufweist, die so ausgelegt sind, dass sie die Konzentration roter Blutkörperchen in dem Blut, das in dem Sammelbehältnis (1) enthalten ist, durch Erfassung in dem Behältnis (1) oder in dem Kanal (2), der das Sammelbehältnis (1) mit dem Behältnis (3) zur Infusion an den Patienten bzw. direkt mit dem Patienten verbindet, erfassen, und die so ausgelegt sind, dass sie Befestigungsmittel (14, 15, 16 und 17) betätigen, die so ausgelegt sind, dass sie den Kanal (2) am Ausgang des Sammelbehältnisses (1) selektiv schließen und öffnen, so dass die Passage des Flüssigkeitsstromes durch ihn hindurch blockiert bzw. ermöglicht wird, wobei die Erfassungsmittel (9, 10), die so ausgelegt sind, dass sie die Konzentration roter Blutkörperchen in dem Blut, das in dem Sammelbehältnis (1) enthalten ist, erfassen, so ausgelegt sind, dass sie den Grad der Absorption einer das Blut durchlaufenden sichtbaren Strahlung messen, wobei die Vorrichtung (4) weiterhin ein Gehäuse (5) mit einer Passung, die die Verbindung der Vorrichtung (4) mit dem Kanal (2) am Ausgang des Blutsammelbehältnisses (1) ermöglicht, umfasst, **dadurch gekennzeichnet, dass** die Erfassungsmittel (9, 10), die so ausgelegt sind, dass sie die Konzentration roter Blutkörperchen in dem von dem Kanal (2) transportierten Blutstrom erfassen, in das Gehäuse (5) eingesetzt sind, mit einer in dem Gehäuse (5) befindlichen Management-Karte (11) verbunden sind und eine Quelle (9) einer sichtbaren Strahlung umfassen, die so angeordnet ist, dass die Strahlung durch den Kanal (2) läuft und an einem Empfänger (10) aus ihm austritt, wobei das Gehäuse (5) weiterhin in seinem Inneren die Befestigungsmittel enthält, die Folgendes umfassen: einen Elektromotor (13), der durch die Management-Karte (11) gesteuert wird und so ausgelegt ist, dass er ein exzentrisches Auflager (14) betätigt, das mit Hilfe eines Keils an der Abgangswelle des Motors (13) befestigt ist und eine Walze (15) zwischen einer ersten Hubgrenzenposition, in der die Walze (15) den Kanal (2) gegen ein Widerlager (16) zusammendrückt, bis sie seine komplette Schließung bewirkt, und einer zweiten Hubgrenzenposition, in der die Walze (15) keinen Kontakt mit dem Kanal (2) hat und sich dieser daher im offenen Zustand befindet, stützt.

2. Vorrichtung nach Anspruch 1, **dadurch gekennzeichnet, dass** das Gehäuse (5) eine Tür (7) umfasst, die sich zwischen einer offenen Position, die eine Verbindung des Gehäuses (5) mit dem Kanal (2) am Ausgang des Blutsammelbehältnisses (1) ermöglicht, und einer geschlossenen Betriebsposition bewegt.

3. Vorrichtung nach Anspruch 1 oder 2, **dadurch gekennzeichnet, dass** die Walze (15), die mit dem von dem Elektromotor (13) betätigten exzentrischen Auflager (14) verbunden ist, den Kanal (2) zum Transport des Blutes gegen ein elastisches Widerlager (17) zusammendrückt.

4. Vorrichtung nach einem der vorangegangenen Ansprüche, **gekennzeichnet durch** das Vorhandensein eines Hubbegrenzers für die Bewegung des Elektromotors zur Betätigung des exzentrischen walzenauflagers (14), der zwei Nocken (18, 19) umfasst, die mit der Abgangswelle (13a) des Motors (13) verstellbar verbunden sind und in Kombination mit Mikroschaltern (18a, 19a), die mit der Management-Karte (11) in dem Gehäuse (5) der Vorrichtung verbunden sind, arbeiten.

5. Vorrichtung nach einem der Ansprüche 2 bis 4, **dadurch gekennzeichnet, dass** die Tür (7) mittels Angeln an dem Gehäuse der Vorrichtung befestigt ist und folgende Mittel zur Arretierung in einer geschlossenen Position aufweist: eine Stange (20), die innerhalb einer Passung in der Tür (7) axial gleiten kann und durch die Wirkung eines elastischen Mittels (22) in der unteren Hubgrenzenposition gehalten wird, in der sie mittels einer Taste (23) aus der unteren Vorderseite der Tür (7) herausragt, wobei die Stange (20) in einer Zwischenposition eine Aussparung (24) aufweist, die so ausgelegt ist, dass sie sich passgenau in eine Zugriffsnut (25) für den Zugriff auf eine für die Aufnahme der Stange (20) konzipierte Passung (26), die in einer mit dem Gehäuse (5) gemeinsam verbundenen Platte (27) ausgebildet ist, einfügt, wobei die Nut (25) von einer geneigten Fläche (28) flankiert ist, die so ausgelegt ist, dass sie mit einer entsprechenden geneigten Fläche (29) auf der Stange (20) am oberen Rand der Aussparung (24) in Kontakt kommt, so dass die Stange (20) während der Schließbewegung der Tür (7) an der Nut (25) bis zum Einschnappen in die Passung (26) infolge der Wirkung des elastischen Mittels (22) angehoben und die Tür (7) demzufolge geschlossen wird.

## Revendications

1. Dispositif pour contrôler l'administration d'érythrocytes à un patient dans une ligne de transfusion, ladite ligne de transfusion comprenant un réservoir (1) de collecte du sang raccordé, au moyen d'un tube (2), soit à un réservoir (3) pour transfusion au patient, soit directement au patient, et ledit dispositif (4) étant équipé de moyens de détection (9, 10) qui sont aptes à détecter la concentration en érythrocytes dans le sang contenu dans le réservoir de collecte (1) par une détection effectuée sur ledit réservoir (1) ou sur le tube (2) qui relie ledit réservoir de collecte (1) au réservoir (3) pour la transfusion au patient ou directement au patient, et qui sont aptes à mettre en oeuvre des moyens de serrage (14, 15, 16, 17) qui sont aptes à fermer et ouvrir sélectivement ledit tube (2) en sortie du réservoir de collecte (1) de manière à respectivement bloquer ou permettre le passage du flux de liquide à travers celui-ci, lesdits moyens de détection (9, 10) aptes à détecter la concentration en érythrocytes dans le sang contenu dans le réservoir de collecte (1) étant aptes à mesurer le degré d'absorption d'un rayonnement lumineux qui traverse le sang, le dispositif (4) comprenant en outre un corps (5) pourvu d'un siège qui permet d'associer ledit dispositif (4) au tube (2) en sortie du réservoir (1) de collecte du sang, ***caractérisé en ce que*** lesdits moyens de détection (9, 10) aptes à détecter la concentration en érythrocytes dans le flux sanguin acheminé par ledit tube (2) sont insérés dans ledit corps (5), sont reliés à une carte de gestion (11) qui est reçue à l'intérieur dudit corps (5) et comprennent une source (9) d'un rayonnement lumineux qui est placée de telle sorte que ledit rayonnement traverse ledit tube (2) et en ressorte au niveau d'un récepteur (10), ledit corps (5) comportant en outre intérieurement lesdits moyens de serrage qui comprennent un moteur électrique (13) contrôlé par la carte de gestion (11) et apte à actionner un support excentrique (14) calé sur l'arbre de sortie du moteur (13) et qui supporte un rouleau (15) entre une première position de fin de course, dans laquelle le rouleau (15) comprime le tube (2) contre une butée (16) jusqu'à ce qu'il provoque sa fermeture totale, et une deuxième position de fin de course, dans laquelle le rouleau (15) est hors de contact avec ledit tube (2) qui est par conséquent dans la position ouverte.

2. Dispositif selon la revendication 1, ***caractérisé en ce que*** le corps (5) comprend une trappe (7) qui peut se déplacer entre une position ouverte, qui permet d'associer ledit corps (5) au tube (2) en sortie du réservoir (1) de collecte du sang, et une position de fonctionnement fermée.

3. Dispositif selon la revendication 1 ou 2, ***caractérisé en ce que*** le rouleau (15) associé au support excentrique (14) actionné par le moteur électrique (13) comprime le tube (2) pour acheminer le sang contre une butée élastique (17).

4. Dispositif selon l'une ou plusieurs des revendications précédentes, ***caractérisé par*** la présente d'un limiteur de course du mouvement du moteur électrique pour actionner le support excentrique (14) du rouleau qui comprend deux cames (18, 19) qui sont associées de manière réglable avec l'arbre de sortie (13a) du moteur (13) et fonctionnent en combinaison avec des microrupteurs (18a, 19a) associés à la carte de gestion (11) contenue dans le corps (5) du dispositif.

5. Dispositif selon l'une ou plusieurs des revendications 2 à 4, ***caractérisé en ce que*** ladite trappe (7) est articulée au corps du dispositif et est pourvue de moyens de blocage dans une position de fermeture, qui comprend une tige (20) qui peut coulisser axialement dans un siège qui est formé dans la trappe (7) et est maintenue par l'action de moyens élastiques (22) dans la position de fin de course inférieure, dans laquelle elle fait saillie par un bouton (23) de la face inférieure de ladite trappe (7), ladite tige (20) étant pourvue, dans une position intermédiaire, d'un retrait (24) qui est apte à être inséré en force dans une encoche d'accès (25) pour accéder à un siège (26) conçu pour recevoir ladite tige (20), encoche formée dans une plaque (27) qui est solidaire dudit corps (5), ladite encoche (25) étant flanquée d'un plan incliné (28) qui est apte à venir en contact avec un plan incliné correspondant (29) qui est placé sur la tige (20) sur le rebord supérieur du retrait (24), de manière à soulever la tige (20) pendant le mouvement de fermeture de la trappe (7) au niveau de l'encoche (25) jusqu'à une insertion à déclic dans son siège (26) produite par l'action des moyens élastiques (22), avec verrouillage consécutif de la trappe (7).
